# EUROPEAN PATENT SPECIFICATION

(11) **EP 1 266 967 B1**
(45) Date of publication and mention of the grant of the patent: **28.01.2004**
(21) Application number: 01114462.3
(22) Date of filing: 15.06.2001
(51) Int. Cl.: C12P 7/62, C12P 7/42, C12P 17/06

(54) **Process for the production of pravastatin and lovastatin**
Verfahren zur Herstellung von Pravastatin und Lovastatin
Procédé de production de pravastatine et de lovastatine

(43) Date of publication of application: 18.12.2002
(73) Proprietor: GNOSIS SRL, 21050 Cairate (Varese) (IT)
(72) Inventor: Benedetti, Alberto, 20063 Cernusco sul Naviglio (MI) (IT); Manzoni, Matilde, 20090 Segrate - Milano 2 (IT); Nichele, Marina, 20020 Vanzaghello (MI) (IT); Rollini, Manuela, 20094 Corsico (MI) (IT)
(74) Representative: Mancini, Vincenzo, Dr.

(56) References cited:
- DATABASE CA [Online] CHEMICAL ABSTRACTS SERVICE, COLUMBUS, OHIO, US; MANZONI, MATILDE ET AL: "Production of statins by filamentous fungi" retrieved from STN Database accession no. 131:43626 XP002191877 & BIOTECHNOL. LETT. (1999), 21(3), 253-257,

## Description

The invention relates to a process for the production of pravastatin. Particularly, the invention relates to a fermentation process for the production of exocellular pravastatin, directly in a fermentation medium, by cultivation of microorganisms from *Monascus* species.

Statins are fungal secondary metabolites which inhibit hydroxy-methylglutaryl coenzime A (HMG-CoA) reductase (EC 1.1.1.34) as the first committed enzyme of cholesterol biosynthesis (Alberts et al. , Am. J. Cardiol., 62: 10J-15J, 1988). Statins are therefore used as cholesterol-lowering drugs. All statins possess a common structure, a hexahydronaphthalene system and a β-hydroxylactone; their differences are due to side chains and methyl groups around the ring. Lovastatin has a methylbutyryl side-chain at 8-α and a methyl group in 6-α of the naphthalene ring; mevastatin lacks the methyl group; pravastatin is the 6-hydroxy sodium salt analogue of mevastatin; monacolin J lacks the lovastatin methylbutyryl side-chain.

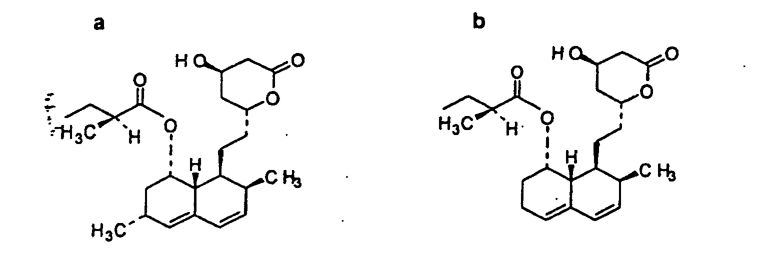

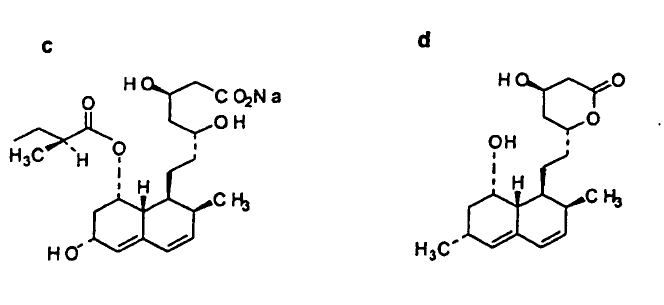

Lovastatin (a), mevastatin (b), pravastatin (c) and monacolin J (d) structures.

Endo et al. (J. Antibiot. 29: 1346-1348, 1976 and FEBS 72: 323-326, 1976) described a process for the production and purification of mevastatin from *Penicillin citrinum* (NRRL 8082). After this, lovastatin was obtained from a number of cultures of *Monascus ruber* (Negishi *et al*., Hakko Kogaku Kaishi, 64: 509-512, 1986) and, in 1989, an industrial process for its production was set up using *Aspergillus terreus* (ATCC 20542) which yielded nearly 180 mg lovastatin/l, glycerol being the carbon source in a fedbatch culture (Buckland *et al.* in: "Novel microbial product for Medicine and Agriculture" by A. L. Demain et al., 161-169, Elsevier, 1989).

Manzoni *et al.* (Biotechnology Techniques 12: 7, 529-532, 1998) described a process for the production of statins from *Aspergillus terreus* (MIM A1 and A2 strains, Industrial Microbiology Collection of the University of Milan, Italy), in which these metabolites were partly in the culture medium. Manzoni *et al.* again (Biotechnology Letters, 21: 253-257, 1999) described a process for the production of lovastatin, pravastatin and monacolin J from *Monascus paxii* AM12M (Industrial Microbiology Collection of the University of Milan, Italy), a spontaneous mutant, in which 64% of lovastatin and 53% of pravastatin were in the medium. According to Manzoni *et al*. in the latter article, the total production yields of the strains they selected were as per Table 1 hereunder:

**Table 1. -**

| - Lovastatin and pravastatin yields (mg/l) and recovery from *A. terreus* (BST strain, Industrial Microbiology Collection of the University of Milan, Italy) and *M. paxii* (AM12M) cultures. | | | | |
|---|---|---|---|---|
| Strain | Lovastatin | | Pravastatin | |
| | (mg/l)* | (%) | (mg/l)* | (%) |
| *Aspergillus terreus* BST | | | | |
| culture filtrate | 36 | 17 | 94 | 50 |
| mycelium | 224 | 83 | 93 | 50 |
| *Monascus paxii* AM12M | | | | |
| culture filtrate | 100 | 64 | 34 | 53 |
| mycelium | 56 | 36 | 30 | 47 |

| | | | | |
|---|---|---|---|---|
| * Yields are referred to 1 l culture filtrate and to the corresponding recovered mycelium. | | | | |

Although these processes already represent an improvement for the production of pravastatin and lovastatin, their production yields are relatively low. Thus, higher yields and lower costs still represent an important need for producing both pravastatin and lovastatin.

In our laboratories, a mutated strain of the microorganism *Monascus ruber* ATCC 22080 was isolated and identified in our strain collection as GN/33, filed on June 19, 2000 with the DSMZ, identified with the number DSM 13554.

### Characteristics of the Monascus ruber DSM 13554

A mutated strain of *Monascus ruber* ATCC 22080 was isolated from a culture on agar as sectorial mutant of colony, macromorphologically different from the starting strain.

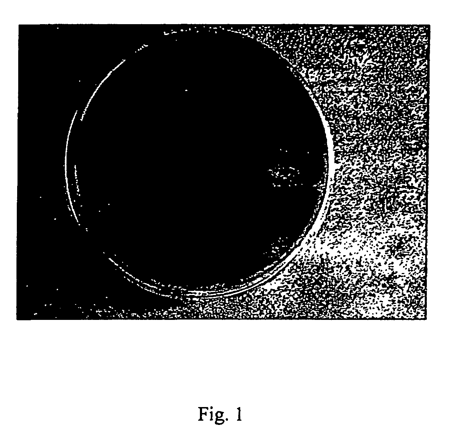

Figure 1 shows a colony of *Monascus ruber* ATCC 22080, wherein a morphologically different sector appears; said sector was then isolated and stabilised in order to avoid regression. The isolated *Monascus ruber*, with respect to *Monascus ruber* ATCC 22080, showed a different pigmentation during the sporification phase while no differences in structure morphology were evidenced in microscopic observation.

### Production of pravastatin

It has now been found, according to a first aspect of the present invention, that pravastatin can be produced by means of a fermentation process which comprises:
a) pre-fermenting *Monascus ruber* DSM 13554 (GN/33) strain, in aerobic conditions, in a first aqueous nutrient medium comprising at least one assimilable source of carbon and nitrogen;
b) fermenting the pre-fermented strain in aerobic conditions, in a second aqueous nutrient medium comprising at least one assimilable source of carbon and nitrogen and
c) recovering pravastatin.

Particularly, at least one of the above media comprises a mineral salt and steps a) and b) are carried out for 2 to 4 days, preferably 3 days, and 4 to 12 days, preferably 9 days, respectively, both at a temperature ranging from 20 to 35°C, preferably 22 to 28°C, and a pH ranging from 4 to 8, preferably from 5 to 7.

The process of the invention may further be advantageously carried out by aerating at least one of the pre-fermenting and fermenting steps with either air or oxygen gas and/or mixture thereof.

The process of the invention allows to obtain exocellular pravastatin, either in a cell-associated form or releasable into the culture broth, directly, as a secondary metabolite, in a fermentation culture medium, with production yields in a very high level, i.e. 1 to 4.0 g/l, using *Monascus ruber* DSM 13554 (GN/33).

The above mentioned first and second aqueous nutrient media have to be different from one another. The above mentioned two microorganisms are cultivated in culture media comprising at least one simple and/or complex carbon source and at least one organic and/or inorganic nitrogen source; preferably at least one mineral salt is added to at least one of the media.

The simple and complex carbon sources can come, for instance, from agricultural and/or industrial wastes; particularly, they can comprise at least one of the following substances: sugars, such as dextrose, dextrin, glucose, lactose, fructose, saccharose, mannitol, mannose; organic acids; alcohols; aldehydes; glycerol; starches such as maize and potato starch; fats; oils; hydrocarbons and whey and the like.

The organic and inorganic nitrogen sources can comprise, for instance, at least one of the following substances: malt extract, corn steep liquor, casein enzymatic hydrolysate, soya flours, cotton flours, arachis flours, dry yeast extracts, peptones such as soy peptone, meat extracts, nitrates, amino acids, casein, ammonium salts such as ammonium sulphate, urea, ammonia and the like.

The mineral salts preferably used in the process of the invention can vary depending on the culture medium; the soluble inorganic salts which provide sodium, potassium, ammonium, magnesium, calcium, copper, sulphate, chloride, nitrate, carbonate ions, can be used. Such salts can be, for instance, sodium nitrate, copper sulphate, ammonium nitrate, monobasic and bibasic potassium phosphate, magnesium sulphate, monobasic potassium sulphate, sodium nitrate, calcium carbonate.

Further, the medium may comprise at least an amino acid (e.g. cysteine, methionine, glutammate, glutamine, glicine, leucine, etc.) and/or a phosphorus source (e.g., potassium phosphate, etc.) and/or an alcohol (e.g. methanol, ethanol, etc.)

Both the pre-fermenting and the fermenting steps are carried out either in flask or in fermenter, according to the traditional methods, for a time depending on the microorganism according to what above illustrated and as the skilled in the art would easily understand, through the passage from slant to pre-seed, seed and fermenter.

The production of pravastatin was followed through analytical assays during the fermentation course.
Analytical methods
HPLC chromatography
Instrument: Merck Hitachi
L-7100 pump; L-7400 detector; L-7200 Autosampler
Injection volume: 20 microliters
Column Merck Lichrospher 60 RP Select B (5 microliter), room temperature
Mobile phase: acetonitrile/water acidified with trifluoracetic acid (0.05% v/v) (40:60), flow 0.8 ml/min⁻¹
Detector: UV 230 nm; absorbance sensitivity 0.050.

### Sample preparation:

After centrifugation (5 min. at 10,000 rpm) of the mycelium as it is and/or washed, the clear upper phase (100 microliters) was adequately diluted and injected into the HPLC system.

### Quantification:

A standard amount of pravastatin was dissolved in methanol with a concentration of 300 mg/l. The samples were tested with variable concentrations, ranging between 50 and 300 mg/l.

The recovery step (step c)] is carried out according to the traditional methods, as the skilled in the art would easily understand, for instance by means of filtration of the mycelium and extraction of the raw product, at the end of fermentation, from the sole upper phase with an organic solvent such as, for instance, ethyl acetate, methylene chloride, chloroform, acetonitrile, acetone, petroleum ether and the like.

The purification of the extracted fermentation product may be carried out by means of chromatography on ionexchange resins and/or absorption resins such as, for instance RELITE 2AS and SEPABEADS SP 207.

The following examples illustrate the invention without limiting it.

### Example 1

The strain *Monascus ruber* GN/33 - DSM 13554, was stored in agar-slant consisting of PDA and incubated for 10 days at 25°C.

250 ml flasks were used, each containing 50 ml of the pre-culture medium having the following composition (g/l) in 1000 ml tap water:

| | |
|---|---|
| Glucose | 25 |
| Casein hydrolysate | 10 |
| Yeast extract | 15 |
| NaNO₃ | 2 |
| MgSO₄·7 H₂O | 0.5 |
| NaCl | 1 |

The sterilization was carried out at 121°C for 20 min. yet, glucose was sterilized separately and added to the culture medium later on under sterile conditions; the resulting pH of 4.4 was adjusted to 6.2 with 1N NaOH.

Each flask was inoculated with 1.5 ml of a standard suspension, in sterile distilled H₂O, containing Tween 0.5%, spores (absorbance 0.1 at 580 nm) obtained from a culture in PDA incubated at 25°C for 2 days. The flasks were placed in incubation at 28°C on an alternative shaker (60 strokes/min, 5 cm run).

1000 ml flasks equipped with baffle were used, each containing 100 ml of culture medium with the following composition (g/l) in 1000 ml tap water:

| | |
|---|---|
| Glycerol | 60 |
| Glucose | 20 |
| Peptone | 10 |
| Whole soy flour | 30 |
| NaNO₃ | 2 |
| MgSO₄·7 H₂O | 0.5 |

The sterilization was carried out at 121°C for 30 min. yet, glucose was sterilized separately and added to the culture medium later on under sterile conditions; the resulting pH of 5.9 was adjusted to 5.8 with 1N HCl.

Each flask was inoculated with 10% of the 2 days pre-culture. The flasks were placed in incubation at 25°C on alternative shaker (60 strokes/min, 5 cm run) for 144 hours.

At the end of fermentation, the culture was analysed with the following result: production of pravastatin 1435 mg/l.

### Example 2

The procedure illustrated in Example 1 was repeated yet, the flask was inoculated (10%) with a broth-culture of 48 hours, obtained using the same culture medium and under the same fermentation conditions as those reported in Example 4.

At the end of fermentation (120 hours), the culture was analysed with the following result: production of pravastatin 1025 mg/l.

### Example 3

The spores of *Monascus ruber* DSM 13554 were collected, as described in Example 1, and inoculated in 750 ml flasks equipped with baffle, containing 100 ml of culture medium having the following composition (g/l) in 1000 ml tap water:

| | |
|---|---|
| Glycerol | 40 |
| Glucose | 20 |
| Peptone | 10 |
| Whole soy flour | 10 |
| NaNO₃ | 2 |
| MgSO₄·7 H₂O | 0.5 |

The sterilization was carried out at 121°C for 30 min. yet, glucose was sterilized separately and added to the culture medium later on under sterile conditions; the resulting pH of 5.7 was adjusted to 5.8 with 1N NaOH.

The flasks were inoculated and incubated as described in Example 1, for 72 hours.

A fermenter of useful volume of 5 1 was prepared, containing the culture medium with the following composition (g/l) in 1000 ml tap water:

| | |
|---|---|
| Glycerol | 65 |
| Glucose | 30 |
| Peptone | 10 |
| Defatted soy flour, Cargill type | 25 |
| NaNO₃ | 2 |
| MgSO₄·7 H₂O | 0.5 |

The sterilization was carried out at 121°C for 45 min. yet, glucose was sterilized separately and added to the culture medium later on under sterile conditions; the resulting pH of 5.8 was adjusted to 5.5 with 1N HCl.

The fermenter was inoculated with the 72 hours pre-fermented broth-culture coming from 5 flasks, as above described (inoculum 10%).

Fermentation was performed under the following conditions: temperature 25°C, stirring 300 rpm, aeration 1.5 vvm, addition of 8 ml antifoam (Polypropylene glycol 2000, Aldrich).

At the end of fermentation (96 hours), the culture was analysed, with the following result: production of pravastatin 3065 mg/l.

Isolation of pravastatin was performed by means of filtration of the mycelium and extraction from the sole upper phase with solvents. The culture filtered mass was acidified at pH 1.5 with trifluoracetic acid, keeping temperature between 0° and 5°C. Extraction was performed using an equal volume of ethyl acetate and performing extraction twice. The two extracted volumes were collected, dehydrated with sodium sulphate anhydrous, filtered and concentrated under vacuum, thus obtaining a total extraction yield of 85% (2605 mg/l, raw product).

The raw mass was treated with H₂O and absorbed on strong anionic resin RELITE 2AS (Resindion S.R.L.-Mitsubishi Chemical Corporation); elution with sodium acetate was performed.

A second absorption was performed on adsorbent resin SEPBEADS SP 207 (Resindion S.R.L.-Mitsubishi Chemical Corporation); the mass is eluted with H₂O and lyophilized (yield: 1302,5 mg/l).

### Example 4

The procedure illustrated in Example 3 was repeated yet, methylene chloride was used as solvent to carry out the extraction.

An extraction yield of 65% (1992 mg/l of raw pravastatin) of fermentation was obtained.

### Example 5

The procedure illustrated in Example 3 was repeated yet, after filtration, the mycelium was washed with acidulated H₂O and then extracted as reported in Example 3, thus obtaining a pravastatin yield of 1700 mg/l.

### Example 6

The procedure illustrated in Example 3 was repeated yet, the harvest medium was microfiltered and washed with acidulated water. The filtered limpid was extracted as described in Example 3 obtaining 4183 mg/l of raw pravastatin.

## Claims

1. A fermentation process for producing pravastatin which comprises
a) pre-fermenting *Monascus ruber* DSM 13554 (GN/33) strain, in aerobic conditions, in a first aqueous nutrient medium comprising at least one assimilable source of carbon and nitrogen;
b) fermenting the pre-fermented strain in aerobic conditions, in a second aqueous nutrient medium comprising at least one assimilable source of carbon and nitrogen and
c) recovering pravastatin.

2. A fermentation process according to claim 1, wherein at least one medium comprises a mineral salt.

3. A fermentation process according to any of the previous claims, wherein steps a) and b) are carried out for 2 to 4 and 4 to 12 days, respectively, at a temperature ranging from 20 to 35°C and a pH ranging from 4 to 8.

4. Process according to any of the previous claims, wherein steps a) and b) are carried out for 3 and 9 days, respectively, at a temperature ranging from 22 to 28°C and a pH ranging from 5 to 7.

5. Process according to any of the previous claims, wherein the carbon source comprises at least one of the following substances: sugars, organic acids, alcohols, aldehydes, glycerol, starches, fats, oils, hydrocarbons and whey.

6. Process according to any of the previous claims, wherein the carbon source comprises at least one of the following substances: dextrose, dextrin, glucose, lactose, fructose, saccharose, mannitol, mannose, maize starch and potato starch.

7. Process according to any of the previous claims, wherein the nitrogen source comprises at least one of the following substances: malt extract, corn steep liquor, casein enzymatic hydrolysate, soya flours, cotton flours, arachis flours, dry yeast extracts, peptones, meat extracts, nitrates, amino acids, casein, ammonium salts, urea, ammonia.

8. Process according to any of the previous claims, wherein at least one medium comprises a mineral soluble inorganic salt which provides sodium, potassium, ammonium, magnesium, calcium, copper, sulphate, chloride, nitrate, carbonate ions.

9. Process according to any of the previous claims, wherein at least one medium comprises at least one of the following salts: sodium nitrate, copper sulphate, ammonium nitrate, monobasic and bibasic potassium phosphate, magnesium sulphate, monobasic potassium sulphate, sodium nitrate, calcium carbonate.

10. Process according to any of the previous claims, wherein step a) and/or step b) are carried out by aeration with either air or oxygen gas and/or mixture thereof.

## Patentansprüche

1. Fermentations-Verfahren zur Herstellung von Pravastatin, umfassend:
a) Vorfermentieren vom *Monascus Ruber*-Stamm DSM 13554 (GN/33) unter aeroben Bedingungen in einem ersten wässrigen Nährmedium, das mindestens eine assimilierbare Quelle für Kohlenstoff und Stickstoff enthält;
b) Fermentieren des vorfermentierten Stammes unter aeroben Bedingungen in einem zweiten wässrigen Nährmedium, dass mindestens eine assimilierbare Quelle für Kohlenstoff und Stickstoff umfasst, und
c) Gewinnen von Pravastatin.

2. Fermentations-Verfahren nach Anspruch 1, wobei mindestens ein Medium ein Mineralsalz umfasst.

3. Fermentations-Verfahren nach einem der vorstehenden Ansprüche, wobei Schritte a) und b) über 2 bis 4 bzw. 4 bis 12 Tage bei einer Temperatur im Bereich von 20 bis 35 °C und einem pH im Bereich von 4 bis 8 durchgeführt werden.

4. Verfahren nach einem der vorstehenden Ansprüche, wobei Schritte a) und b) über 3 bzw. 9 Tage bei einer Temperatur im Bereich von 22 bis 28°C und einem pH im Bereich von 5 bis 7 durchgeführt werden.

5. Verfahren nach einem der vorstehenden Ansprüche, wobei die Kohlenstoff-Quelle mindestens eine der folgenden Substanzen umfasst: Zucker, organische Säuren, Alkohole, Aldehyde, Glycerin, Stärken, Fette, Öle, Kohlenwasserstoffe und Molke.

6. Verfahren nach einem der vorstehenden Ansprüche, wobei die Kohlenstoff-Quelle mindestens eine der folgenden Substanzen umfasst: Dextrose, Dextrin, Glukose, Laktose, Fruktose, Saccharose, Manitol, Mannose, Maisstärke und Kartoffelstärke.

7. Verfahren nach einem der vorstehenden Ansprüche, wobei die Stickstoffquelle mindestens eine der folgenden Substanzen umfasst: Malzextrakt, Maisquellwasser, enzymatisches Casein-Hydrolysat, Sojamehle, Baumwollmehle, Arachismehle, trockene Hefeextrakte, Peptone, Fleischextrakte, Nitrate, Aminosäuren, Casein, Ammoniumsalze, Harnstoff, Ammoniak.

8. Verfahren nach einem der vorstehenden Ansprüche, wobei mindestens ein Medium ein lösliches anorganisches Mineralsalz umfasst, welches Natrium-, Kalium-, Ammonium-, Magnesium-, Calcium-, Kupfer-, Sulfat-, Chlorid-, Nitrat-, Carbonat- Ionen bereitstellt.

9. Verfahren nach einem der vorstehenden Ansprüche, wobei mindestens ein Medium mindestens eines der folgenden Salze umfasst: Natriumnitrat, Kupfersulfat, Ammoniumnitrat, einbasisches und zweibasisches Kaliumphosphat, Magnesiumsulfat, einbasisches Kaliumsulfat, Natriumnitrat, Calciumcarbonat.

10. Verfahren nach einem der vorstehenden Ansprüche, wobei Schritt a) und/oder Schritt b) unter Begasung entweder mit Luft oder Sauerstoff-Gas und/oder einem Gemisch daraus durchgeführt werden.

## Revendications

1. Procédé de fermentation pour la production de pravastatine qui comprend :
(a) la pré-fermentation de la souche *Monascus ruber* DSM 13554 (GN/33) dans des conditions aérobies, dans un premier milieu nutritif aqueux comportant au moins une source assimilable de carbone et d'azote ;
(b) la fermentation de la souche pré-fermentée dans des conditions aérobies dans un second milieu nutritif aqueux comportant au moins une source assimilable de carbone et d'azote ;
(c) la récupération de la pravastatine.

2. Procédé de fermentation selon la revendication 1, dans lequel un milieu au moins comprend un sel minéral.

3. Procédé de fermentation selon l'une quelconque des revendications précédentes, dans lequel les étapes a) et b) sont réalisées, respectivement, en 2 à 4 jours et en 4 à 12 jours, à une température dans la plage de 20 à 35 °C et à un pH compris entre 4 et 8.

4. Procédé selon l'une quelconque des revendications précédentes, dans lequel les étapes a) et b) sont réalisées, respectivement, en 3 et 9 jours, à une température dans la plage de 22 à 28 °C et à un pH compris entre 5 et 7.

5. Procédé selon l'une quelconque des revendications précédentes, dans lequel la source de carbone comprend au moins l'une des substances suivantes : sucres, acides organiques, alcools, aldéhydes, glycérol, amidons, graisses, huiles, hydrocarbures et petit lait.

6. Procédé selon l'une quelconque des revendications précédentes, dans lequel la source de carbone comprend au moins l'une des substances suivantes : dextrose, dextrine, glucose, lactose, fructose, saccharose, mannitol, amidon de maïs et amidon de pomme de terre.

7. Procédé selon l'une quelconque des revendications précédentes, dans lequel la source d'azote comprend au moins l'une des substances suivantes : extrait de malt, eau de trempage, hydrolysat enzymatique de caséine, farine de soja, farine de coton, farine d'arachide, extraits secs de levure, peptones, extraits de viande, nitrates, acides aminés, caséine, sels d'ammonium, urée, ammoniac.

8. Procédé selon l'une quelconque des revendications précédentes, dans lequel un milieu au moins comprend un sel inorganique minéral soluble fournissant du sodium, du potassium, de l'ammonium, du magnésium, du calcium, du cuivre, des ions sulfate, chlorure, nitrate, et carbonate.

9. Procédé selon l'une quelconque des revendications précédentes, dans lequel un milieu au moins comprend l'un des sels suivants : nitrate de sodium, sulfate de cuivre, nitrate d'ammonium, phosphate monobasique et dibasique de potassium, sulfate de magnésium, sulfate monobasique de potassium, nitrate de sodium, carbonate de calcium.

10. Procédé selon l'une quelconque des revendications précédentes, dans lequel l'étape a) et/ou l'étape b) sont réalisées sous une aération soit d'air soit d'oxygène et/ou un mélange de ceux-ci.


### SEQUENCE LISTING

<110> GNOSIS s.r.l.
<120> PROCESS FOR THE PRODUCTION OF PRAVASTATIN
<130> G68359VM/GF
<160> 4
<170> PatentIn version 3.1
<210> 1
<211> 20
<212> DNA
<213> Aspergillus terreus
<400> 1
   gcatcgatga agaacgcagc 20
<210> 2
<211> 20
<212> DNA
<213> Aspergillus terreus
<400> 2
   tcctccgctt attgatatgc 20
<210> 3
<211> 341
<212> DNA
<213> Aspergillus terreus
<400> 3
   gaatgcgata actaatgtga attgcagaat tcagtgaatc atcgagtctt tgaacgcaca 60
   ttgcgccccc tggtattccg gggggcatgc ctgtccgagc gtcattgctg ccctcaagcc 120
   cggcttgtgt gttgggccct cgtcccccgg ctcccggggg acgggcccga aaggcagcgg 180
   cggcaccgcg tccggtcctc gagcgtatgg ggcttcgtct tccgctccgt aggcccggcc 240
   ggcgcccgcc gacgcattta tttgcaactt gtttttttcc aggttgacct cggatcaggt 300
   agggataccc gctgaactta agcatatcaa taagcggagg a 341
<210> 4
<211> 344
<212> DNA
<213> Aspergillus terreus
<400> 4
   cttaagttca gcgggtatcc ctacctgatc cgaggtcaac ctggaaaaaa acaagttgca 60
   aataaatgcg tcggcgggcg ccggccgggc ctacggagcg gaagacgaag ccccatacgc 120
   tcgaggaccg gacgcggtgc cgccgctgcc tttcgggccc gtcccccggg agccggggga 180
   cgagggccca acacacaagc cgggcttgag ggcagcaatg acgctcggac aggcatgccc 240
   cccggaatac cagggggcgc aatgtgcgtt caaagactcg atgattcact gaattctgca 300
   attcacatta gttatcgcat ttcgctgcgt tcttcatcga tgca 344